# EUROPEAN PATENT SPECIFICATION

(11) **EP 2 985 027 B1**
(45) Date of publication and mention of the grant of the patent: **31.03.2021**
(21) Application number: 14181205.7
(22) Date of filing: 16.08.2014
(51) Int. Cl.: A61K 31/728, A61K 33/14, A61K 33/04, A61K 45/06

(54) **NASAL COMPOSITION COMPRISING MIXTURE OF HYALURONIC ACIDS AND SALINE SOLUTION**
NASENHEILMITTEL ENTHALTEND EIN GEMISCH VON HYALURONSÄUREN UND SALZLÖSUNG
COMPOSITION NASALE RENFERMANT UN MÉLANGE D'ACIDES HYALURONIQUES ET UNE SOLUTION SALINE

(43) Date of publication of application: 17.02.2016
(73) Proprietor: Church & Dwight Co., Inc., Princeton, NJ 08543-5297 (US)
(72) Inventor: Saaid, Amina, 60870 Rieux (FR)
(74) Representative: August Debouzy

(56) References cited:
- EP-A1- 1 847 256
- US-A1- 2004 071 740
- US-A1- 2005 164 979
- US-A1- 2010 222 308
- US-A1- 2014 010 847
- US-A1- 2014 141 077
- MACCHI A ET AL: "Hyaluronan plus saline nasal washes in the treatment of rhino-sinusal symptoms in patients undergoing functional endoscopic sinus surgery for rhino-sinusal remodeling.", INTERNATIONAL JOURNAL OF IMMUNOPATHOLOGY AND PHARMACOLOGY 2013 JAN-MAR, vol. 26, no. 1, January 2013 (2013-01), pages 137-145, XP009180658, ISSN: 0394-6320
- CANTONE ELENA ET AL: "Impact of intranasal sodium hyaluronate on the short-term quality of life of patients undergoing functional endoscopic sinus surgery for chronic rhinosinusitis.", INTERNATIONAL FORUM OF ALLERGY & RHINOLOGY JUN 2014, vol. 4, no. 6, June 2014 (2014-06), pages 484-487, XP002730900, ISSN: 2042-6984

## Description

### FIELD OF THE INVENTION

The present invention refers to nasal compositions, containing a film-forming agent.

### TECHNICAL BACKGROUND

Nasal sprays are known in the art.

The applicant is manufacturing nasal sprays currently on the market under the trademark Sterimar®. Three main grades are available, as follows. The copper formulation is dedicated to blocked nose, the manganese formulation is dedicated to allergic nose and the sulfur composition is dedicated to irritated nose.

It is also known to use in a nasal spray a specific grade of hyaluronic acid. FR2847818 discloses the use of a low MW hyaluronic acid, with a MW below 10⁵ Daltons. It is also indicated that the mucociliary clearance is improved with low MWs compared to high MWs.

Document US 2004/071740 discloses a colloidal pharmaceutical preparation useful in the treatment of diseases of the upper and lower respiratory tract, and lung parenchyma, comprising as an active ingredient, a colloidal mixture of hyaluronic acids of different molecular weights.

Document US 2014/141077 discloses a composition of hyaluronic acids in combination with a drug for treating and preventing an inflammation related disorder.

Document US 2014/010847 discloses a composition of hyaluronic acids for treating and preventing mucosa related disorders or diseases wherein the symptoms include ulceration, inflammation, allergic reaction and bleeding.

Yet, the compositions of the prior art still require improvements, notably for the antiviral activity and the film-forming activity.

### SUMMARY OF THE INVENTION

The invention provides a nasal composition comprising:
- a saline base comprising sodium chloride;
- an active agent comprising copper, a mixture of copper and manganese, manganese, a combination of manganese and a calcium salt, or sulfur;
- a mixture of a first hyaluronic acid with a second hyaluronic acid.

According to the invention, the first hyaluronic acid has a molecular weight of at most 5.10⁴ Da, preferably between 20 000 and 50 000.

According to the invention, the second hyaluronic acid has a molecular weight of at least 100 000 daltons and at most 500 000 daltons, preferably between 100 000 and 300 000.

According to one embodiment, the first and second hyaluronic acids are present according to a weight ratio of 10:1 to 1:10, preferably from 5:1 to 1:5, more preferably from 3:1 to 1:3, more advantageously from 2:1 to 1:2 and especially about 1:1.

According to one embodiment, the first and second hyaluronic acids are each present in amounts from 0.01 to 1 g, preferably from 0.05 to 0.5 g, more preferably from 0.1 to 0.3 g of hyaluronic acid per 100 ml of nasal composition.

According to one embodiment, the composition is hypotonic, isotonic or hypertonic, preferably isotonic or hypertonic.

According to one embodiment, the active agent is copper or a mixture of copper and manganese, preferably as metallic salt(s), and the nasal composition is an hypertonic composition.

According to one embodiment, the active agent is manganese, preferably as metallic salt, optionally in combination with a calcium salt, and the nasal composition is an hypertonic composition.

According to one embodiment, the active agent is sulfur, preferably as salt, and the nasal composition is an isotonic composition.

According to one embodiment, the composition further comprises an alkyl poly glucoside, preferably selected from the list consisting of decyl glucoside, coco glucoside, lauryl glucoside, hexyl glucoside, butyl glucoside, caprylyl/capryl glucoside and mixtures thereof, more preferably in combination with a copper salt.

According to one embodiment, the nasal composition is a spray.

The invention also discloses the composition of the invention for its use for the prophylactic or therapeutic treatment of diseases of the upper airways, preferably by pulverization or spraying (notably into the nose) of said composition to a patient in need thereof.

The invention also relates to a dispenser, preferably equipped with a pump, containing the nasal composition of the invention.

The compositions of the invention thus:
- encapsulates foreign particles (allergens, dust, pollution, etc.) responsible for the allergic reaction, inactivates and removes them quickly ;
- provides a protective, imperceptible, film on the nasal mucosa which protects it from further attacks, soothes and hydrates it ;
- significantly strengthens the barrier function in full respect of the mucosa.

The invention also allows for improved anti-viral efficacy.

The invention further provides advantages as will be apparent from the description.

### BRIEF DESCRIPTION OF THE DRAWINGS

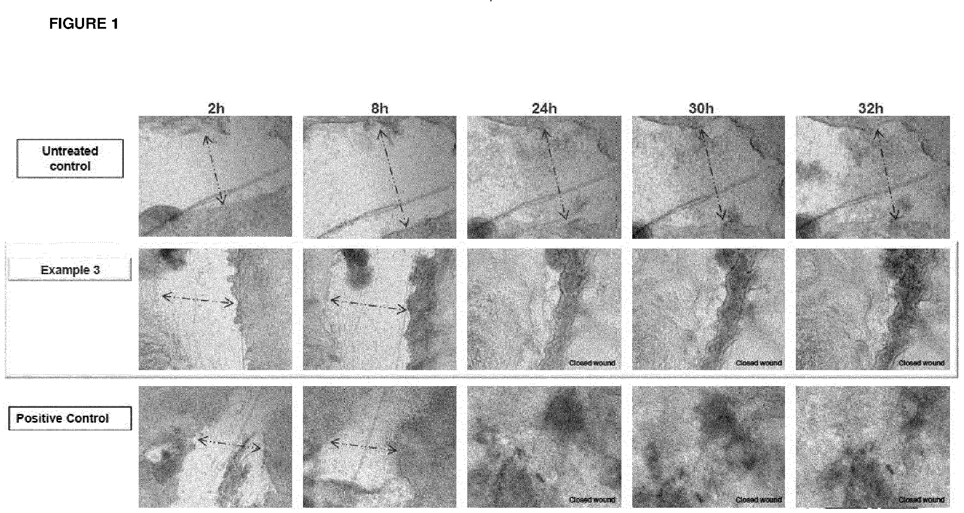

- Figure 1 is a series of microphotographs re-epithelization for a untreated control, example 3 and positive control.

### DESCRIPTION OF EMBODIMENTS OF THE INVENTION

The invention is now disclosed in more details in the following description. Unless specified otherwise, amounts and percentages are by weight. Molecular weights are expressed in Daltons (or kilodaltons).

### Saline base.

The saline base is the one typically used in nasal compositions. The composition containing the saline base can be hypotonic, isotonic or hypertonic (including slightly hypertonic), depending on the specific use that is intended for the nasal spray. The salt used in the saline base can be the typical sea salts or sodium chloride, since the saline composition typically comprises filtered sea water, but other salts can be used. For example, calcium chloride salt can be used.

Preferred compositions are isotonic (i.e containing about 32 ml of sea water without added salt, for a total volume of aqueous composition of 100 ml) or are hypertonic, notably with 45 to 80 ml sea water per 100 ml, preferably from 50 to 75 ml sea water per 100 ml of composition.

### Active agent

An active ingredient is typically used in the nasal sprays. A metallic salt is typically used in a nasal spray, such as a copper salt and/or a manganese salt. Sulfur is also used in nasal sprays, notably as thio compounds. The Applicant is already marketing nasal sprays containing said agent, where each agent is more specifically dedicated to one specific pathology or symptom. In summary, the copper formulation is dedicated to blocked nose, the manganese formulation is dedicated to allergic nose and the sulfur composition is dedicated to irritated nose.

This is already in use in the commercial Sterimar® products, marketed by the Applicant, as recalled above.

The respective amounts can be varied according to the extent of treatment, the duration, the volume sprayed into the nose, and the like. It has been found that the amounts used in the existing commercial compositions are appropriate for the respective treatments.

A drug can be present as well. Possible drugs are disclosed in WO01/93846, see page 5, lines 3-13. Further possible drugs are also disclosed in WO2006/003521, at paragraphs [0081] to [0094]. A steroid or an antihistamine can for example be present.

A formulation with copper will preferably be hypertonic.

A formulation with manganese will preferably be (slightly) hypertonic.

A formulation with sulfur will preferably be isotonic.

### Hyaluronic compound

The hyaluronic compound of the invention is a mixture of two grades of hyaluronic acid (HA). The term hyaluronic acid also encompasses salts and derivatives thereof, especially sodium hyaluronate.

The first part is the low molecular weight (low MW) hyaluronic acid disclosed in FR2847818. It can be prepared according to the general processes of fermentation, as is recalled in said document FR2847818.

The low MW hyaluronic acid has a molecular weight at most 5.10⁴ Da, preferably between 20 000 and 50 000.

It is also available on the market by the supplier Soliance, under the tradename PrimalHyal 50/Renovhyal.

The second part is the medium molecular weight (medium MW) hyaluronic acid. It can be prepared according to the general biotechnological processes.

The medium MW hyaluronic acid has a molecular weight at least 10⁵ Da, and at most 5.10⁵ Da, preferably between 100 000 and 300 000.

It is also available on the market by the supplier Soliance, under the tradename PrimalHyal 300/Bashyal.

The amount of each of the low and medium MW hyaluronic acid can be from 0.01 to 1 g, preferably from 0.05 to 0.5 g, more preferably from 0.1 to 0.3 g of hyaluronic acid (low MW or medium MW) per 100 ml of composition of the invention.

The weight ratio of low MW to medium MW HA is from 10:1 to 1:10, preferably from 5:1 to 1:5, more preferably from 3:1 to 1:3, more advantageously from 2:1 to 1:2 and especially about 1:1.

The specific use of two different HAs with different MWs allows an improved film-forming capacity for the composition, and provides the advantages as evidenced in the examples section.

### Alkyl poly glucoside

Alkyl poly glucoside (APG) is a known surfactant. It is a non-ionic surfactant generally of vegetal origin (synthetic origin is also possible), which can be advantageously from renewable materials. It can be selected from the list consisting of decyl glucoside, coco glucoside, lauryl glucoside, hexyl glucoside, butyl glucoside, caprylyl/capryl glucoside (the later being mixed C₈-C₁₀ chain), and mixtures thereof.

The composition comprising the APG (especially in association with the HA) has an anti-viral effect.

The alkyl poly glucoside used in the present invention can be present in amounts from 0.01 to 1, preferably from 0.05 to 0.5 g, more preferably from 0.1 to 0.3 g of alkyl poly glucoside per 100 ml of nasal composition.

### Other ingredients

Any other ingredient commonly used can be present. For example, essential oils can be incorporated if it is so wished. Surfactants, viscosity modifiers, preservatives, coloring agents and the like can also be present, albeit this is not especially preferred.

### Dispensing device.

The composition of the invention can be placed in an appropriate dispensing device for it to be dispensed, preferably as a nasal spray. Preferably a pump system is used, so as to avoid propellants. The use of specific pumps further allows to have preservative-free compositions; it is however possible to have compositions with a preservative. Metered dosing pumps are preferred, where the typical volume of metered dispensed composition is at least 100µl, typically 140µl. The volume of the housing can be for example less than 100ml, typically about 20ml.

However, any type of dispensing devices is appropriate including pressurized vessels using pressuring gas (such as nitrogen). Typical sprays dispensing devices, such as bag-on-valve.

### Therapeutical uses and pharmaceutical compositions.

The composition of the invention is useful for the treatment of a great variety of diseases of the upper airways, for example inflammatory, infectious or allergic rhinitis, acute or chronic inflammatory bronchitis (infectious, allergic or toxic), chronic obstructive pulmonary disease or cystic fibrosis, rhinopharyngitis, cold, bronchiectasia, mucoviscidose, asthma and the like.

The viruses which can be treated thanks to the invention are very diverse. They can be found for example in WO2006/003521, at paragraphs [0049] to [0060]. The invention is especially applicable to the treatment of rhinovirus infection, e.g. an acute or chronic rhinovirus infection. The rhinovirus infection can be in an individual being a high-risk patient selected from the group consisting of an asthma patient, a person suffering from allergy, and a person suffering from an inflammatory disease.

In a preferred embodiment the pharmaceutical composition is adapted for topical or mucosal use and is for example available as sprays, foams, or liquid solutions such as skin lotions, gargle, solutions or nose drops, preferably as nose drops and nasal sprays.

Usually, the composition will be provided as a non-pyrogenous, sterile preparation. In case of a liquid preparation sterility may be achieved, for example, by filtration through a suitable membrane filter. Methods for the manufacture of sterile or aseptic pharmaceutical compositions are well known in the art.

A dose would typically be two pulverizations into each nostril, for example up to 4 to 10, preferably 6 times per day, and generally up to 4 to 10 consecutive days, typically up to 6 days. The typical content of a dose is about 140µl.

The invention also discloses compositions and methods for the prophylactic or therapeutic treatment of diseases of the upper airways, preferably a rhinovirus infection. Notably the method comprises the steps of administering, preferably by pulverization or spraying in the nose, a composition of the invention to a patient in need thereof.

### EXAMPLES

The following examples illustrate the invention without limiting it.

### Example 1 -« blocked nose » composition (anti-viral composition)

The following composition is prepared by adding to the existing Sterimar® "blocked nose" composition the following ingredients:

| Ingredient | Unit formula |
|---|---|
| APG | 0.1-0.3 g |
| HA (20-50kDa) | 0.1-0.3 g |
| HA (100-300kDa) | 0.1-0.3 g |

This composition will :
- Decongests rapidly
- Induce a protective effect at the tight junctions level
- Improve the mucociliary clearance
- Stimulate the phagocytic activity
- Exhibit anti-bacterial potential against *Staphylococcus Aureus*
- Inhibit the replication of HRV providing for prophylactic activity and preventing and fighting infection and surinfection

### Example 2 - « allergic nose » composition

The following composition is prepared by adding to the existing Sterimar® "allergic nose" composition the following ingredients:

| Ingredient | Unit formula |
|---|---|
| Sea water | qsp 50.00 ml |
| HA (20-50kDa) | 0.1-0.3 g |
| HA (100-300kDa) | 0.1-0.3 g |

This composition will :
- Induce a protective effect at the tight junctions level
- Improve the mucociliary clearance
- Stimulate the phagocytic activity

### Example 3 - « irritated nose » composition

The following composition is prepared by adding to the existing Sterimar® "irritated nose" composition the following ingredients:

| Ingredient | Unit formula |
|---|---|
| HA (20-50kDa) | 0.1-0.3 g |
| HA (100-300kDa) | 0.1-0.3 g |

This composition will :
- Induce a protective effect at the tight junctions level
- Have an healing effect on injured epithelium
- Allow a re-epithelialization in full respect of the barrier structure without pharmacological effect.

Each of the three formulations is submitted to various tests, as reported below, evidencing the benefits of the various formulations.

### Cell integrity, cytotoxicity and irritation study.

A 3D model of the human airway epithelia is used as a sensitive and reliable model for in-vitro toxicology and pharmaco-toxicological testing. It is a human nasal epithelium which is reconstituted and is available from the company Epithelix under the tradename MucilAir™. The test provides for TEER (Trans Epithelial Electrical Resistance) measurement (known method to functionally analyze tight junction dynamics in cell culture models of barriers and permeability of epithelium), Resazurin test, Mucociliary clearance and morphology.

### Rezasurin cytotoxicity assay.

This cytotoxicity assay results establish that all formulations show no cytotoxicity and no irritation. For untreated cultures, the value is at 100%. The same value is achieved for all examples 1, 2 and 3.

### Membrane integrity test.

Membrane integrity monitoring is determined using Lactate DeHydrogenase (LDH), which is an enzyme found extensively in body tissues, normally present into the cell (Cytosol) and released during tissue damage. It is a marker of common injuries and disease; the measure of LDH reflects the state of cells: its absence in the extracellular medium equating membrane integrity.

The LDH assay results establish tissue integrity at day 1, 2, 3 and 4. This is apparent from assays comparing the formulations of examples 1, 2 and 3 with saline solution and untreated cultures. None of them exhibit any cytotoxicity as measured using LDH as a marker.

### Pro-inflammatory effect test.

The airway epithelia is a physical barrier and modulator of immune responses and its cells synthesize and release a large panel of inflammatory mediators (cytokines and chemokines) upon external pathogen/chemical challenge. Interleukin 8 (IL-8) is one of the most abundant cytokines released by the airway epithelial cells after inflammatory stimuli. It is a reliable biomarker for detecting the adverse effects of the chemicals. It is a good marker for allergic response.

The IL-8 assay results establish tissue integrity at day 1, 2, 3 and 4. This is apparent from the below table, comparing the formulations of examples 1, 2 and 3 with saline solution and untreated cultures as well as a positive control. The following table provides the values expressed in ng/ml, at days 1, 2, 3 and 4

| | IL-8 D1 | IL-8 D2 | IL-8 D3 | IL-8 D4 |
|---|---|---|---|---|
| Untreated cultures | 5.6 | 8.6 | 12.0 | 14.6 |
| Saline Solution | | 6.7 | 14.5 | 32.8 |
| Example 1 | 10.1 | 33.4 | 19.4 | 11.4 |
| Example 2 | 11.8 | 9.8 | 10.0 | 8.7 |
| Example 3 | 5.2 | 4.5 | 8.9 | 6.0 |
| Positive control | 201.1 | 268.3 | 264.8 | 278.0 |

### Film-forming efficacy of example 1 composition

The film-forming efficacy is deduced by the measurement of the TEER at T=0, then at T=72h, compared to untreated control.

An increase in the value is evidencing the film-forming capability of the tested composition. The result is given in the table graph below where the saline solution is the negative control and 100% is the arbitrary value given at T=0; results are expressed as %.

| | Saline solution | Example 1 |
|---|---|---|
| T=0h | 100 | 100 |
| T=72h | 93 | 223 |

### Mucociliary clearance of example 1 composition

The mucociliary clearance is an important primary innate defense mechanism that protects the lungs from deleterious effects of inhaled pollutants, allergens, and pathogens in the nose; mucus is moved toward the pharynx by cilia beating.

The result is given in the table below which provides the mucociliary clearance at days 1 and 4, expressed in µm/s, for the untreated cultures, the saline solution, the composition of the example and a positive control.

| | MCC D1 | MCC D4 |
|---|---|---|
| Untreated cultures | 20.42 | 20.73 |
| Saline Solution | 49.22 | 39.06 |
| Example 1 | 50.34 | 50.53 |
| Positive control | 54.50 | 48.32 |

### Phagocytosis of example 1 composition.

Phagocytosis is a cellular process of foreign particle ingestion:
- The virus, bacteria, allergens or foreign particle is engulfed
- Internalized in a vesicle into the cell (Phagosome)
- Usually the content of this vesicle is destroyed and eliminated.

The phagocytosis assay is based on latex fluorescent micro-beads (as foreign particles) after 1 h treatment with the product.

The tissue treated for 1h with the products induced an increase in fluorescence intensity expressed as RFU (relative fluorescence unit) compared to the control cells (with saline solution) corresponding to an increase in phagocytic activity for example 1 composition. The result is given in the table below.

| | Control | Example 1 |
|---|---|---|
| RFU | 13405 | 44016 |
| Fold increase | 1 | 3.3 |

Allergens and viruses would be more rapidly eliminated, reducing the risks of negative effect of allergens and viruses.

### Anti-bacterial activity of example 1 composition.

Monitoring of cultivable Staphylococcus aureus densities after tested strains seeding in the formulations allows determination of the bactericidal effect. The table below evidences that the composition exhibits a bactericidal effect from 1 hour, by measuring the densities expressed in UFC/ml for times of 1, 3 and 24 hours.

| | 0 | 1 | 3 | 24 |
|---|---|---|---|---|
| Growth control | 3.2 10⁶ | 4.4 10⁶ | 2.2 10⁷ | 5.10 10⁹ |
| Example 1 | 3.2 10⁶ | 1 | 1 | 1 |

### Anti-viral effect of example 1 composition.

The example 1 composition is tested according to the test of evaluation of the potential activity against HRV-A16 (a rhinovirus responsible for colds) on fully differentiated human airway epithelial cells culture. The compositions are first applied before infection for one hour and the replication is carried out for 4 hours. 3 rinsing steps with the formulations are carried out. The remaining is collected and the RNA is dosed after cell lysis. The same collecting step is carried out 24 hours after and the same dosing step is carried out. The percentage expressed the changes in viral RNA, resulting in the % of inhibition of HRV-A16 replication, leading to the anti-viral efficacy. The tested composition is tested against rupintrivir (a known antiviral compound, providing an almost complete response). The results are the following:

| Product | Inhibition (%) |
|---|---|
| Rupintrivir | 99.62 |
| Example 1 formulation | 99.8 |

### Film-forming efficacy of example 2 composition

The film-forming efficacy is deduced by the measurement of the TEER at T=0, then at T=72h, compared to untreated control.

An increase in the value is evidencing the film-forming capability of the tested composition. The result is given in the table graph below where the saline solution is the negative control and 100% is the arbitrary value given at T=0; results are expressed as %.

| | Saline solution | Example 2 |
|---|---|---|
| T=0h | 100 | 100 |
| T=72h | 93 | 127 |

### Mucociliary clearance of example 2 composition

The mucociliary clearance is an important primary innate defense mechanism that protects the lungs from deleterious effects of inhaled pollutants, allergens, and pathogens in the nose, mucus is moved toward the pharynx by cilia beating.

The result is given in the table below which provides the mucociliary clearance at days 1 and 4, expressed in µm/s, for the untreated cultures, the saline solution, the composition of the example and a positive control.

| | MCC D1 | MCC D4 |
|---|---|---|
| Untreated cultures | 20.42 | 20.73 |
| Saline Solution | 49.22 | 39.06 |
| Example 2 | 34.85 | 61.29 |
| Positive control | 54.50 | 48.32 |

### Phagocytosis of example 2 composition.

Phagocytosis is a cellular process of foreign particle ingestion:
- The virus, bacteria, allergens or foreign particle is engulfed
- Internalized in a vesicle into the cell (Phagosome)
- Usually the content of this vesicle is destroyed and eliminated.

The phagocytosis assay is based on latex fluorescent micro-beads (as foreign particles) after 1 h treatment with the product.

The tissue treated for 1h with the products induced an increase in fluorescence intensity expressed as RFU (relative fluorescence unit) compared to the control cells (with saline solution) corresponding to an increase in phagocytic activity for example 2 composition. The result is given in the table below.

| | Control | Example 2 |
|---|---|---|
| RFU | 13405 | 16271 |
| Fold increase | 1 | 1.2 |

Allergens would be more rapidly eliminated, reducing the risks of negative effect of allergens.

### Film-forming efficacy of example 3 composition

The film-forming efficacy is deduced by the measurement of the TEER at T=0, then at T=72h, compared to untreated control.

An increase in the value is evidencing the film-forming capability of the tested composition. The result is given in the table graph below where the saline solution is the negative control and 100% is the arbitrary value given at T=0; results are expressed as %.

| | Saline solution | Example 3 |
|---|---|---|
| T=0h | 100 | 100 |
| T=72h | 113 | 146 |

### Re-epithelialization activity of example 3 composition.

The example 3 composition induced a strong front of proliferation and migration at the edge of the gap (visible difference between 8h and 24h). At 24h the wound is completely closed. The healing activity of the example 3 composition is similar to a positive control.

This is visible from the comparison of the microphotographs of figure 1.

The increase in occludin suggests its involvement in stabilizing the tight junction maintaining the barrier integrity helping the re-epithelialization process. The increase in integrin β1 facilitates the migration of epithelial cells into the wounded area by organizing the cell cytoscheleton. This is apparent from the table below, including a positive control and evidencing the efficacy of the composition; the saline solution (negative control) being arbitrarily set at the value of 1 and the other value being relative. There is a wound healing due to a mechanical effect (substantially without pharmacological effect), since the composition stimulates both occludin and integrin β1, resulting in cells migration rather than proliferation as would do a drug.

| Sample | Occludin | Integrin β1 |
|---|---|---|
| Saline solution | 1 | 1 |
| Injured epithelia | 1.1745 | 1.37 |
| Example 3 | 1.933 | 2.105 |
| Positive control | 0.912 | 1.446 |

## Claims

1. Nasal composition comprising:
- a saline base comprising sodium chloride;
- an active agent comprising copper, a mixture of copper and manganese, manganese, a combination of manganese and a calcium salt, or sulfur;
- a mixture of a first hyaluronic acid with a second hyaluronic acid;
wherein the first hyaluronic acid has a molecular weight of at most 50 000 daltons; and
wherein the second hyaluronic acid has a molecular weight of at least 100 000 daltons and at most 500 000 daltons.

2. Nasal composition of claim 1, wherein the first hyaluronic acid has a molecular weight in the range of 20 000 and 50 000 daltons.

3. Nasal composition of claim 1 or 2, wherein the second hyaluronic acid has a molecular weight in the range of 100 000 and 300 000 daltons.

4. Nasal composition of any one of claims 1 to 3, wherein the first and second hyaluronic acids are present according to a weight ratio of 10:1 to 1:10, preferably from 5:1 to 1:5, more preferably from 3:1 to 1:3, more advantageously from 2:1 to 1:2 and especially about 1:1.

5. Nasal composition of any one of claims 1 to 4, wherein the first and second hyaluronic acids are each present in amounts from 0.01 to 1 g, preferably from 0.05 to 0.5 g, more preferably from 0.1 to 0.3 g of hyaluronic acid per 100 ml of nasal composition.

6. Nasal composition of any one of claims 1 to 5, wherein the composition is hypotonic, isotonic or hypertonic, preferably isotonic or hypertonic.

7. Nasal composition of any one of claims 1 to 6, wherein the active agent is copper or a mixture of copper and manganese, as metallic salt(s), and wherein the nasal composition is a hypertonic composition.

8. Nasal composition of any one of claims 1 to 6, wherein the active agent is manganese, as metallic salt, optionally in combination with a calcium salt, and wherein the nasal composition is a hypertonic composition.

9. Nasal composition of any one of claims 1 to 6, wherein the active agent is sulfur, as salt, and wherein the nasal composition is an isotonic composition.

10. Nasal composition of any one of claims 1 to 9, further comprising a alkyl poly glucoside, preferably selected from the list consisting of decyl glucoside, coco glucoside, lauryl glucoside, hexyl glucoside, butyl glucoside, caprylyl/capryl glucoside and mixtures thereof, more preferably in combination with a copper salt.

11. Nasal composition of any one of claims 1 to 10, which is a spray.

12. Nasal composition of any one of claims 1 to 11, for the prophylactic or therapeutic treatment of diseases of the upper airways, preferably by pulverization or spraying of said composition to a patient in need thereof.

13. The nasal composition of any one of claims 1 to 12, wherein the saline base is a sea salt.

14. A dispenser, preferably equipped with a pump, containing the nasal composition of any one of the preceding claims.

## Patentansprüche

1. Nasale Zusammensetzung, die umfasst:
- eine salinische Base, die Natriumchlorid umfasst;
- einen Wirkstoff, der Kupfer, eine Mischung aus Kupfer und Mangan, Mangan, eine Kombination aus Mangan und einem Calciumsalz oder Schwefel umfasst;
- eine Mischung aus einer ersten Hyaluronsäure mit einer zweiten Hyaluronsäure;
wobei die erste Hyaluronsäure ein Molekulargewicht von höchstens 50.000 Dalton aufweist; und
wobei die zweite Hyaluronsäure ein Molekulargewicht von zumindest 100.000 Dalton und höchstens 500.000 Dalton aufweist.

2. Nasale Zusammensetzung nach Anspruch 1, wobei die erste Hyaluronsäure ein Molekulargewicht im Bereich von 20.000 und 50.000 Dalton aufweist.

3. Nasale Zusammensetzung nach Anspruch 1 oder 2, wobei die zweite Hyaluronsäure ein Molekulargewicht im Bereich von 100.000 und 300.000 Dalton aufweist.

4. Nasale Zusammensetzung nach einem der Ansprüche 1 bis 3, wobei die erste und die zweite Hyaluronsäure in einem Gewichtsverhältnis von 10:1 bis 1:10, vorzugsweise 5:1 bis 1:5, mehr bevorzugt 3:1 bis 1:3, vorteilhafter 2:1 bis 1:2 und insbesondere ungefähr 1:1 vorhanden sind.

5. Nasale Zusammensetzung nach einem der Ansprüche 1 bis 4, wobei die erste und die zweite Hyaluronsäure jeweils in Mengen von 0,01 bis 1 g, vorzugsweise 0,05 bis 0,5 g, mehr bevorzugt von 0,1 bis 0,3 g, Hyaluronsäure pro 100 ml nasale Zusammensetzung vorhanden sind.

6. Nasale Zusammensetzung nach einem der Ansprüche 1 bis 5, wobei die Zusammensetzung hypotonisch, isotonisch oder hypertonisch, vorzugsweise isotonisch oder hypertonisch, ist.

7. Nasale Zusammensetzung nach einem der Ansprüche 1 bis 6, wobei der Wirkstoff Kupfer oder eine Mischung aus Kupfer und Mangan als metallisches Salz bzw. metallische Salze ist und wobei die nasale Zusammensetzung eine hypertonische Zusammensetzung ist.

8. Nasale Zusammensetzung nach einem der Ansprüche 1 bis 6, wobei der Wirkstoff Mangan als metallisches Salz ist, optional in Kombination mit einem Calciumsalz, und wobei die nasale Zusammensetzung eine hypertonische Zusammensetzung ist.

9. Nasale Zusammensetzung nach einem der Ansprüche 1 bis 6, wobei der Wirkstoff Schwefel als Salz ist und wobei die nasale Zusammensetzung eine isotonische Zusammensetzung ist.

10. Nasale Zusammensetzung nach einem der Ansprüche 1 bis 9, ferner umfassend ein Alkylpolyglucosid, das vorzugsweise aus der Auflistung ausgewählt ist, die aus Decylglucosid, Cocoglucosid, Laurylglucosid, Hexylglucosid, Butylglucosid, Caprylyl/Caprylglucosid und Mischungen davon, mehr bevorzugt in Kombination mit einem Kupfersalz, ausgewählt ist.

11. Nasale Zusammensetzung nach einem der Ansprüche 1 bis 10, bei der es sich um einen Spray handelt.

12. Nasale Zusammensetzung nach einem der Ansprüche 1 bis 11 zur prophylaktischen oder therapeutischen Behandlung von Erkrankungen der oberen Atemwege, vorzugsweise durch Pulverisieren oder Sprühen der Zusammensetzung auf einen Patienten, der dies benötigt.

13. Nasale Zusammensetzung nach einem der Ansprüche 1 bis 12, wobei die salinische Base ein Meeressalz ist.

14. Spender, der vorzugsweise mit einer Pumpe ausgestattet ist, der die nasale Zusammensetzung nach einem der vorstehenden Ansprüche enthält.

## Revendications

1. Composition nasale comprenant :
- une base salée comprenant du chlorure de sodium ;
- un principe actif comprenant du cuivre, un mélange de cuivre et de manganèse, du manganèse, une combinaison de manganèse et d'un sel calcique, ou du soufre ;
- un mélange d'un premier acide hyaluronique avec un deuxième acide hyaluronique ;
dans laquelle le premier acide hyaluronique a un poids moléculaire d'au plus 50 000 daltons ; et
dans laquelle le deuxième acide hyaluronique a un poids moléculaire d'au moins 100 000 daltons et d'au plus 500 000 daltons.

2. Composition nasale selon la revendication 1, dans laquelle le premier acide hyaluronique a un poids moléculaire situé dans la plage allant de 20 000 à 50 000 daltons.

3. Composition nasale selon la revendication 1 ou 2, dans laquelle le deuxième acide hyaluronique a un poids moléculaire situé dans la plage allant de 100 000 à 300 000 daltons.

4. Composition nasale selon l'une quelconque des revendications 1 à 3, dans laquelle les premier et deuxième acides hyaluroniques sont présents en un rapport en poids de 10/1 à 1/10, de préférence de 5/1 à 1/5, mieux encore de 3/1 à 1/3, plus avantageusement de 2/1 à 1/2 et tout spécialement d'environ 1/1.

5. Composition nasale selon l'une quelconque des revendications 1 à 4, dans laquelle chacun des premier et deuxième acides hyaluroniques est présent en une quantité de 0,01 à 1 g, de préférence de 0,05 à 0,5 g, mieux encore de 0,1 à 0,3 g d'acide hyaluronique pour 100 ml de composition nasale.

6. Composition nasale selon l'une quelconque des revendications 1 à 5, laquelle composition est hypotonique, isotonique ou hypertonique, de préférence isotonique ou hypertonique.

7. Composition nasale selon l'une quelconque des revendications 1 à 6, dans laquelle le principe actif est le cuivre ou un mélange de cuivre et de manganèse, sous forme de sels métalliques, et laquelle composition nasale est une composition hypertonique.

8. Composition nasale selon l'une quelconque des revendications 1 à 6, dans laquelle le principe actif est le manganèse, sous forme de sel métallique, éventuellement en combinaison avec un sel calcique, et laquelle composition nasale est une composition hypertonique.

9. Composition nasale selon l'une quelconque des revendications 1 à 6, dans laquelle le principe actif est le soufre, sous forme de sel, et laquelle composition nasale est une composition isotonique.

10. Composition nasale selon l'une quelconque des revendications 1 à 9, comprenant en outre un alkylpolyglucoside, de préférence choisi dans la liste consistant en le décylglucoside, le (coprah-yl)glucoside, le laurylglucoside, l'hexylglucoside, le butylglucoside, le caprylyl/caprylglucoside et leurs mélanges, mieux encore en combinaison avec un sel de cuivre.

11. Composition nasale selon l'une quelconque des revendications 1 à 10, qui est un spray.

12. Composition nasale selon l'une quelconque des revendications 1 à 11, pour le traitement prophylactique ou thérapeutique de maladies des voies aériennes supérieures, de préférence par pulvérisation ou projection de ladite composition à un patient en ayant besoin.

13. Composition nasale selon l'une quelconque des revendications 1 à 12, dans laquelle la base salée est le sel de mer.

14. Distributeur, de préférence équipé d'une pompe, contenant la composition nasale de l'une quelconque des revendications précédentes.
